# EUROPÄISCHE PATENTSCHRIFT

(11) **EP 2 219 016 B1**
(45) Veröffentlichungstag und Bekanntmachung des Hinweises auf die Patenterteilung: **27.03.2019**
(21) Anmeldenummer: 09015687.8
(22) Anmeldetag: 18.12.2009
(51) Int. Cl.: G01K 1/08, G01K 1/10, G01K 1/16, G01K 13/02, A61M 1/16

(54) **Temperaturmesseinrichtung**
Temperature measurement device
Dispositif de mesure de la température

(30) Priorität: 12.02.2009 DE 102009008554
(43) Veröffentlichungstag der Anmeldung: 18.08.2010
(73) Patentinhaber: Schunk Kohlenstofftechnik GmbH, 35452 Heuchelheim (DE)
(72) Erfinder: Weck, Rudolf, 35764 Sinn (DE); Gärtner, Ralf, 35633 Lahnau (DE)
(74) Vertreter: advotec.

(56) Entgegenhaltungen:
- CN-A- 1 850 305
- DE-A1- 4 442 427
- GB-A- 1 448 133
- US-A- 4 341 992
- US-A- 4 721 533
- US-A- 5 040 901
- US-A1- 2008 253 427

## Beschreibung

Die vorliegende Erfindung betrifft ein Dialysegerät mit einer Temperaturmesseinrichtung zur Temperaturmessung in einem strömenden oder nicht strömenden fluiden Medium, wobei die Temperaturmesseinrichtung zumindest einen Temperatursensor und ein Sensorgehäuse zur Aufnahme des Temperatursensors aufweist, wobei das Sensorgehäuse zumindest eine in einem Wärmeleitkörper ausgebildete und zu einer Fluidkontaktfläche des Wärmeleitkörpers hin verschlossene Sensoraufnahme aufweist in der der Temperatursensor in wärmeleitendem Kontakt zur Fluidkontaktfläche aufgenommen ist.

Temperaturmesseinrichtungen der eingangsgenannten Art finden in unterschiedlichen Einsatzgebieten Anwendung. Besonders hohe Anforderungen an derartige Temperaturmesseinrichtungen bestehen insbesondere beim Einsatz zur Temperaturmessung auf Gebieten, in denen es vor allem auf die chemische Beständigkeit oder Reinheit der Temperaturmesseinrichtung im Kontaktbereich zum Medium, dessen Temperatur gemessen werden soll, ankommt. Dabei ist die vorgenannte chemische Beständigkeit in zweierlei Hinsicht von Bedeutung. Zum einen kann es darauf ankommen, dass bei der Temperaturmessung in einem chemisch aggressiven Medium die Funktion der Temperaturmesseinrichtung nicht durch unerwünschte Auswirkungen des Mediums auf den Wärmeleitkörper beeinträchtigt wird. Zum anderen kann es sich auch als wesentlich erweisen, dass insbesondere bei einem fluiden Medium keine Migration von schädlichen oder unerwünschten Bestandteilen aus dem Wärmeleitkörper in das Medium stattfindet. Letzteres ist besonders im Bereich der Medizintechnik von Bedeutung, wo Temperaturmesseinrichtungen etwa in einem Dialysegerät zum Einsatz kommen und dort zur Messung der Temperatur des am Wärmeleitkörper der Temperaturmesseinrichtung vorbeiströmenden bzw. den Wärmeleitkörper der Temperaturmesseinrichtung umströmenden Dialysats dienen.

Aufgrund der vorstehend skizzierten Anforderungen hat sich in der Medizintechnik, insbesondere den Bereich der Dialysegeräte betreffend, der Einsatz von Temperaturmesseinrichtungen durchgesetzt, deren Wärmeleitkörper aus Titan besteht. Aufgrund der hohen Rohstoffkosten von Titan als Ausgangsmaterial für den Wärmeleitkörper und wegen der in der Regel eher aufwendigen Materialbearbeitungsverfahren, um Wärmeleitkörper für Temperaturmesseinrichtungen aus Titan herzustellen, sind die bekannten Temperaturmesseinrichtungen zur Verwendung im Bereich der Medizintechnik sehr teuer mit den entsprechenden Auswirkungen auf den Verkaufspreis von Dialysegeräten, die mit derartigen Temperaturmesseinrichtungen bestückt sind.

Aus der US 5 040 901 A ist ein als Herz-Lungen-Maschine ausgeführtes medizinisches Gerät bekannt, das mit einer Temperaturmesseinrichtung versehen ist, die zur Messung einer Temperatur in einer Blutströmung dient. Hierzu weist die Temperaturmesseinrichtung ein als Wärmeleitkörper ausgebildetes Sensorgehäuse auf, in dem ein Temperatursensor in wärmeleitendem Kontakt zu einer Fluidkontaktfläche des Sensorgehäuses aufgenommen ist. Das Sensorgehäuse der Temperaturmesseinrichtung besteht aus einem Metall, wie beispielsweise Titan oder rostfreiem Stahl.

Die US 4 341 992 zeigt eine Temperaturmesseinrichtung, die zum Einführen in Körperöffnungen bestimmt ist. Um eine aus hygienischen Gründen erforderliche Reinigung der Temperaturmesseinrichtung nach deren Verwendung zu erübrigen, ist der Temperatursensor der Temperaturmesseinrichtung in einer austauschbaren wärmeleitfähigen Schutzhülle aufgenommen.

Der vorliegenden Erfindung liegt die Aufgabe zugrunde, ein mit einer Temperaturmesseinrichtung versehenes Dialysegerät vorzuschlagen, dessen Temperaturmesseinrichtung besonders geeignet ist, zur Messung der Temperatur einer Blutströmung.Zur Lösung dieser Aufgabe wird ein Dialysegerät mit den Merkmalen des Anspruchs 1 vorgeschlagen.

Bekanntlich ist Graphit chemisch außerordentlich rein und beständig, so dass ein Einsatz dieses Werkstoffs in der Medizintechnik in unmittelbarem Kontakt zu Körpergewebe oder Körperflüssigkeit unbedenklich ist. Zudem zeichnen sich Graphithaltige Werkstoffe durch einen gute Wärmeleitfähigkeit aus, was deren Verwendung zur Herstellung eines Wärmeleitkörpers besonders vorteilhaft macht.

Erfindungsgemäß wird Elektrographit, polymergebundenen Graphit, metallimprägnierten Graphit, Mesophasengraphit, Metall-Graphit, Graphit-Keramik-Verbundwerkstoffe, Pyrokohlenstoff, Kohlenstofffaserverstärkte Kunststoffe, Kohlenstofffaserverstärkter Kohlenstoff für den Wärmeleitkörper verwendet..

Erfindungsgemäß ist zur Herstellung eines Dialysegerätes mit einer besonders kostengünstigen Temperaturmesseinrichtung bzw. einem besonders kostengünstigen Wärmeleitkörpers der Wärmeleitkörper vollständig aus einem Graphithaltigen Werkstoff hergestellt ist, das darüber hinaus noch homogen ausgebildet ist.

Wenn der Wärmeleitkörper stabförmig ausgebildet ist mit einer zumindest anteilig durch eine Stirnfläche gebildeten Fluidkontaktfläche, ist eine Herstellung besonders einfach möglich, da beispielsweise dieselben Produktionseinrichtungen verwendet werden können, wie sie zur Herstellung von so genannten Kohlebürsten dienen.

Zur Temperaturmessung im Dialysegerät, also bei einer Temperaturmessung in durch Leitungseinrichtungen strömenden Fluiden, ist es von Vorteil, wenn der Wärmeleitkörper ringförmig ausgebildet ist mit einer zumindest anteilig durch eine Innenringfläche gebildeten Fluidkontaktfläche. Dabei kann die Ringausbildung dem Strömungsquerschnitt der Leitungseinrichtung, also beispielsweise der Rohrleitung, angepasst sein und muss nicht unbedingt kreisringförmig sein. Besonders die Möglichkeit der Herstellung von Wärmeleitkörpern in einem Formverfahren eröffnet die Möglichkeit, selbst komplexe polygonale Ringstrukturen zu erzeugen, um eine bestmögliche Anpassung des Wärmeleitkörpers an den Strömungsquerschnitt zu ermöglichen.

Vorteilhaft ist die zumindest eine in einem ringförmigen Wärmeleitkörper ausgebildete Sensoraufnahme radial zur Innenringfläche ausgerichtet, so dass auch Messleitungen zum Anschluss der Messeinrichtung an einen Messverstärker oder dergleichen radial zugeführt werden können. Vorzugsweise ist die Temperaturmesseinrichtung in einer Kanalwandung eines Fluidbehältnisses angeordnet, so dass insbesondere bei einer ringförmigen Ausgestaltung des Wärmeleitkörpers eine im Verhältnis zur Masse des Wärmeleitkörpers sehr große Fluidkontaktfläche möglich wird, was einerseits der Messgenauigkeit zugute kommt, und andererseits Temperaturmesseinrichtungen mit geringer Trägheit ermöglicht, die schon nach sehr kurzer Messzeit genaue Messwerte liefern.

Als besonders vorteilhaft erweist es sich, wenn die Temperaturmesseinrichtung einen ringförmig ausgebildeten Wärmeleitkörper aufweist, der darüber hinaus mit seiner Fluidkontaktfläche flächenbündig in der Kanalwandung angeordnet ist, so dass eine Messung ohne Erzeugung eines Strömungswiderstands möglich wird.

Nachfolgend werden bevorzugte Ausführungsformen der Temperaturmesseinrichtung bzw. einer die Temperaturmesseinrichtung aufweisenden Temperaturmessanordnung anhand der Zeichnung näher erläutert.

Es zeigen:
- **Fig. 1**: eine Temperaturmesseinrichtung mit einem aus einem Grundkörper und einer Beschichtung gebildeten Wärmeleitkörper in perspektivischer Darstellung;
- **Fig. 2**: die in **Fig. 1** dargestellte Temperaturmesseinrichtung in Seitenansicht;
- **Fig. 3**: eine Temperaturmesseinrichtung mit einem als Schichtformkörper ausgebildeten Wärmeleitkörper;
- **Fig. 4**: eine Temperaturmesseinrichtung gemäß einer Ausführungsform der Erfindung mit einem homogen als Graphitkörper ausgebildeten Wärmeleitkörper;
- **Fig. 5**: eine Temperaturmesseinrichtung gemäß einer weiteren Ausführungsform mit einem ringförmig ausgebildeten Wärme leitkörper;
- **Fig. 6**: eine in eine Fluidleitung eingesetzte Temperaturmesseinrichtung.

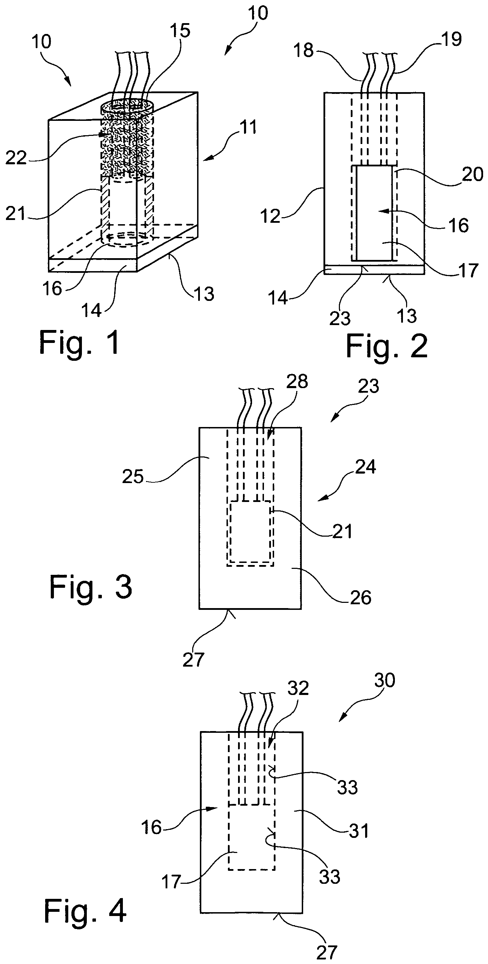

**Fig. 1** zeigt eine Temperaturmesseinrichtung 10 mit einem Wärmeleitkörper 11, der im Falle des vorliegend dargestellten Beispiels aus einem Grundkörper 12 mit einer eine Fluidkontaktfläche 13 ausbildenden, auf dem Grundkörper 12 angeordneten Schicht 14 besteht. Im Grundkörper 12 ist eine hier als Bohrung ausgebildete Sensoraufnahme 15 vorgesehen, in die ein hier als Thermistoreinrichtung ausgebildeter Temperatursensor 16 aufgenommen ist. Die Thermistoreinrichtung weist einen beispielsweise aus Platin gebildeten Sensorkopf 17 auf, der über aus der Sensoraufnahme 15 herausgeführte Messleitungen 18, 19 mit einem hier nicht näher dargestellten Messverstärker verbunden ist, der eine Auswertung der über die Temperaturmesseinrichtung 10 erfassten Messwerte ermöglicht.

Der Temperatursensor 16 bzw. der Sensorkopf 17 ist im vorliegenden Fall über eine einen hier annähernd hülsenförmig ausgebildeten Zwischenraum 20 verfüllende Verbundmasse 21, die beispielsweise aus einem wärmeleitfähigen Kleber bestehen kann, wärmeleitend mit dem Wärmeleitkörper 11 verbunden. Zur hermetischen Abdichtung gegenüber der Umgebung ist der nicht mit dem Sensorkopf 17 ausgefüllte Bereich der Sensoraufnahme 15 vorzugsweise mit einer Dichtmasse 22 verfüllt.

Bei dem in den **Fig. 1** und **2** dargestellten Beispiel der Temperaturmesseinrichtung 10 ist der Grundkörper 12 aus einer Kupferlegierung gebildet, die auf einer Stirnfläche 23 des Grundkörpers 12 mit der Schicht 14 aus Graphit versehen ist. Die Aufbringung bzw. Erzeugung der Schicht 14 kann beispielsweise als Beschichtung über ein Abscheideverfahren, wie beispielsweise CVD (Chemical Vapor Deposition) erfolgen.

**Fig. 3** zeigt eine Temperaturmesseinrichtung 23, die im Unterschied zu der in den **Fig. 1** und **2** dargestellten Temperaturmesseinrichtung 10 einen Wärmeleitkörper 24 aufweist, der als Schichtformkörper hergestellt ist, mit einem aus einer ersten, einen Grundkörper 25 bildenden und aus Kupferpartikeln im Formpressverfahren hergestellten Formteil mit einem unmittelbar daran angeformten, eine Schicht 26 bildenden Formteil aus Graphit. Analog zu der in den **Fig. 1** und **2** dargestellten Temperaturmesseinrichtung 10 wird bei der in **Fig. 3** dargestellten Temperaturmesseinrichtung 23 eine Fluidkontaktfläche 27 durch die Schicht 26 gebildet. Im Unterschied zur Temperaturmesseinrichtung 10 erstreckt sich bei der Temperaturmesseinrichtung 23 eine Sensoraufnahme 28 ausgehend vom Grundkörper 25 bis in die Schicht 26 hinein und quert dabei ein zwischen dem Grundkörper 25 und der Schicht 26 ausgebildeten Übergangsquerschnitt 29. Übereinstimmend mit der Temperaturmesseinrichtung 10 ist in der Sensoraufnahme 28 ein Temperatursensor 16 aufgenommen und vermittels einer Verbundmasse 21 in wärmeleitendem Kontakt mit dem Wärmeleitkörper 24 in der Sensoraufnahme positioniert.

**Fig. 4** zeigt eine Temperaturmesseinrichtung 30 gemäß einem Ausführungsbeispiel der Erfindung, die im Unterschied zur Temperaturmesseinrichtung 10 bzw. zur Temperaturmesseinrichtung 23 einen im Wesentlichen homogen als Graphitkörper ausgebildeten Wärmeleitkörper 31 aufweist. Aufgrund der guten Gleiteigenschaften von Graphitoberflächen ist es möglich, einen Sensorkopf 17 des in einer Sensoraufnahme 32 angeordneten Temperatursensors 16 in unmittelbarem Kontakt mit einer Wandung 33 der Sensoraufnahme 32 in diese einzuführen und an die Wandung 33 angeschmiegt anzuordnen, ohne eine zwischenliegend angeordnete Verbundmasse, wie bei den zuvor erläuterten Temperaturmesseinrichtungen 10 und 23.

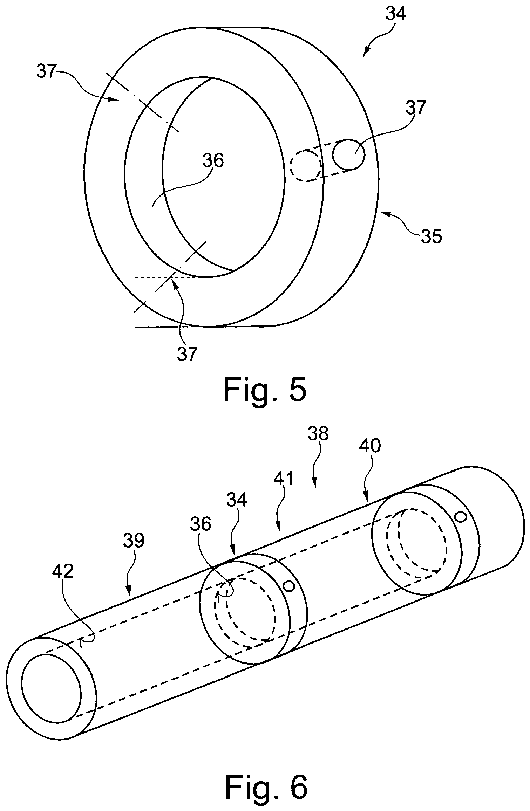

**Fig. 5** zeigt eine Temperaturmesseinrichtung 34 gemäß einem Ausführungsbeispiel der Erfindung, die einen ringförmig ausgebildeten Wärmeleitkörper 35 aufweist, der im Fall des vorliegenden Ausführungsbeispiels homogen aus Graphit ausgebildet ist. Bei der Temperaturmesseinrichtung 34 ist eine Fluidkontaktfläche 36 durch eine innere Mantelfläche des ringförmigen Wärmeleitkörpers 35 gebildet. In dem Wärmeleitkörper 35 befinden sich im Fall des vorliegenden Ausführungsbeispiels mehrere Sensoraufnahmen 37, die jeweils mit hier nicht näher dargestellten Temperatursensoren bzw. zu diesen gehörigen Sensorköpfen entsprechend der Sensoraufnahme 32 der in **Fig. 4** dargestellten Temperaturmesseinrichtung 34 bestückt sind.

**Fig. 6** zeigt die in **Fig. 5** dargestellte Temperaturmesseinrichtung 34 als Teil einer Temperaturmessanordnung 38, die die Temperaturmesseinrichtung 34 zwischen Leitungsteilstücken 39, 40 einer Strömungsleitung 41 aufweist. Dabei ist die Temperaturmesseinrichtung 34 mit ihrer durch die innere Mantelfläche gebildeten Fluidkontaktfläche 36 flächenbündig mit einer Innenwandung 42 der Leitungsteilstücke 39, 40 angeordnet, so dass die Fluidkontaktfläche 36 zwar vollflächig in Kontakt zu dem fluiden Medium steht, dessen Temperatur gemessen werden soll, jedoch keinen Strömungswiderstand in einer durch die Strömungsleitung 41 geleiteten Fluidströmung induziert.

Wie in **Fig. 6** strichpunktiert als optionaler Bestandteil der Temperaturmessanordnung 38 dargestellt, ist es möglich, die Temperaturmessanordnung 38 auch mit einer Mehrzahl Temperaturmesseinrichtungen 34 zu versehen, um beispielsweise auch in einem nicht strömenden Fluidvolumen einen Temperaturgradienten zwischen zwei durch die Temperaturmesseinrichtungen 34 definierten Messstellen ermitteln zu können.

## Patentansprüche

1. Dialysegerät mit einer Temperaturmesseinrichtung (30, 34) zur Temperaturmessung in einem strömenden oder nicht strömenden fluiden Medium, wobei die Temperaturmesseinrichtung zumindest einen Temperatursensor (16) und ein Sensorgehäuse zur Aufnahme des Temperatursensors aufweist, wobei das Sensorgehäuse zumindest eine in einem Wärmeleitkörper (31, 35) ausgebildete und zu einer Fluidkontaktfläche (27, 36) des Wärmeleitkörpers hin verschlossene Sensoraufnahme (32, 37) aufweist, in der der Temperatursensor in wärmeleitendem Kontakt zur Fluidkontaktfläche aufgenommen ist,
**dadurch gekennzeichnet,**
**dass** der Wärmeleitkörper als homogener Körper aus Elektrographit, polymergebundenem Graphit, metallimprägniertem Graphit, Mesophasengraphit, Metall-Graphit, Graphit-Keramik-Verbundwerkstoff, Pyrokohlenstoff, Kohlenstofffaser verstärktem Kunststoff oder Kohlenstofffaser verstärktem Kohlenstoff ausgebildet ist, derart, dass ein Sensorkopf (17) des in der Sensoraufnahme angeordneten Temperatursensors in unmittelbarem Kontakt mit einer Wandung (33) der Sensoraufnahme angeschmiegt angeordnet ist.

2. Dialysegerät nach Anspruch 1,
**dadurch gekennzeichnet,**
**dass** der Wärmeleitkörper (31) stabförmig ausgebildet ist mit einer zumindest anteilig durch eine Stirnfläche gebildeten Fluidkontaktfläche (27).

3. Dialysegerät nach Anspruch 1,
**dadurch gekennzeichnet,**
**dass** der Wärmeleitkörper (35) ringförmig ausgebildet ist mit einer zumindest anteilig durch eine Innenringfläche gebildeten Fluidkontaktfläche (36).

4. Dialysegerät nach Anspruch 3,
**dadurch gekennzeichnet,**
**dass** der Wärmeleitkörper (35) zumindest eine sich radial zur Innenringfläche erstreckende Sensoraufnahme (37) aufweist.

5. Dialysegerät nach einem der vorangehenden Ansprüche,
**dadurch gekennzeichnet,**
**dass** die Temperaturmesseinrichtung in einer Kanalwandung eines Fluidbehältnisses angeordnet ist.

6. Dialysegerät nach Anspruch 5,
**dadurch gekennzeichnet,**
**dass** die Temperaturmesseinrichtung (34) einen ringförmig ausgebildeten und mit seiner Fluidkontaktfläche (36) flächenbündig in der Kanalwandung (42) angeordneten Wärmeleitkörper (35) aufweist.

## Claims

1. A dialysis machine comprising a temperature measuring device (30, 34) for measuring a temperature in a flowing or non-flowing fluid medium, the temperature measuring device having at least one temperature sensor (16) and a sensor housing for housing the temperature sensor, the sensor housing having at least one sensor seat (32, 37) formed in a heat-conducting body (31, 35) and closed toward a fluid contact surface (27, 36) of the heat-conducting body, the temperature sensor being accommodated in said sensor seat in such a manner that it is in heat-conducting contact with the fluid contact surface,
**characterized in that**
the heat-conducting body is a homogenous body made of elec-trographite, polymer-bonded graphite, metal-impregnated graphite, mesophase graphite, metal graphite, graphite/ceramic composite material, pyrocarbon, carbon-fiber-reinforced plastic or carbon-fiber-reinforced carbon in such a manner that a sensor head (17) of the temperature sensor disposed in the sensor seat is nestled in immediate contact to a wall (33) of the sensor seat.

2. The dialysis machine according to claim 1,
**characterized in that**
the heat-conducting body (31) is rod-shaped and has a fluid contact surface (27) formed at least partially by an end face.

3. The dialysis machine according to claim 1,
c**haracterized** in that
the heat-conducting body (35) is ring-shaped and has a fluid contact surface (36) formed at least partially by an inner ring surface.

4. The dialysis machine according to claim 3,
**characterized in that**
the heat-conducting body (25) has at least one sensor seat (37) extending radially to the inner ring surface.

5. The dialysis machine according to any one of the preceding claims,
**characterized in that**
the temperature measuring device is disposed in a channel wall of a fluid container.

6. The dialysis machine according to claim 5,
**characterized in that**
the temperature measuring device (34) has a ring-shaped heat-conducting body (35) whose fluid contact surface (36) is disposed flush in the channel wall (42).

## Revendications

1. Appareil de dialyse comprenant un dispositif (30, 34) de mesure de la température destiné à mesurer la température dans un milieu fluide coulant ou non coulant, le dispositif de mesure de la température comportant au moins un capteur de température (16) et un boîtier de capteur destiné à loger le capteur de température, le boîtier de capteur comportant au moins un logement de capteur (32, 37) formé dans un corps (31, 35) conducteur de chaleur et fermé vers une surface (27, 36) de contact avec le fluide du corps conducteur de chaleur, le capteur de température étant logé en contact conducteur de chaleur avec la surface de contact avec le fluide dans ledit logement de capteur,
**caractérisé en ce que**
le corps conducteur de chaleur est un corps homogène en électrographite, graphite lié a un polymère, graphite imprégné de métal, graphite en mésophase, métal-graphite, matériau composite graphite/céramique, pyrocarbone, plastique renforcé de fibres de carbone ou carbone renforcé de fibres de carbone, de telle manière qu'une tête de capteur (17) du capteur de température disposé dans le logement de capteur se serre en contact direct contre une paroi (33) du logement de capteur.

2. Appareil de dialyse selon la revendication 1,
**caractérisé en ce que**
le corps (31) conducteur de chaleur est en forme de tige et comporte une surface (27) de contact avec le fluide formée au moins partiellement par une face frontale.

3. Appareil de dialyse selon la revendication 1,
**caractérisé en ce que**
le corps (35) conducteur de chaleur est annulaire et comporte une surface (36) de contact avec le fluide formée au moins partiellement par une surface annulaire intérieure.

4. Appareil de dialyse selon la revendication 3,
**caractérisé en ce que**
le corps (35) conducteur de chaleur comporte au moins un logement de capteur (37) s'étendant radialement par rapport à la surface annulaire intérieure.

5. Appareil de dialyse selon l'une quelconque des revendications précédentes,
**caractérisé en ce que**
le dispositif de mesure de la température est disposé dans une paroi de canal d'un récipient de fluide.

6. Appareil de dialyse selon la revendication 5,
**caractérisé en ce que**
le dispositif (34) de mesure de la température a un corps (35) conducteur de chaleur annulaire dont la surface (36) de contact avec le fluide est intégrée à fleur dans la paroi de canal (42).
